# EUROPÄISCHE PATENTSCHRIFT

(11) **EP 0 544 210 B1**
(45) Veröffentlichungstag und Bekanntmachung des Hinweises auf die Patenterteilung: **03.11.1999**
(21) Anmeldenummer: 92119912.1
(22) Anmeldetag: 23.11.1992
(51) Int. Cl.: C07D 403/04, C07D 239/46, C07D 521/00

(54) **Verfahren zur Herstellung von in 5-Stellung substituierten Cytosinen und anderen 4,5-disubstituierten Pyrimidin-2(1H)-onen, sowie dabei auftretende Zwischenprodukte**
Process for the preparation of in position 5 substituted cytosines and of other 4,5-disubstituted pyrimidin-2(1H)-ones, also the forth-issuing intermediates
Procédé de préparation de cytosines substituées en position-5 et d'autres pyrimidines-2-(1H)-ones 4,5-disubstituées, et aussi les intermédiaires se présentant

(30) Priorität: 23.11.1991 DE 4138585
(43) Veröffentlichungstag der Anmeldung: 02.06.1993
(73) Patentinhaber: HOECHST AKTIENGESELLSCHAFT, 65926 Frankfurt am Main (DE)
(72) Erfinder: Jähne, Gerhard, Dr., W-6230 Frankfurt am Main 80 (DE)

(56) Entgegenhaltungen:
- CHEMICAL ABSTRACTS, vol. 100, 1984, Columbus, Ohio, US; abstract no. 209860h, Seite 209853 ;Spalte 2 & PL-A-115 858 (POLFA) 22. Juni 1978
- TETRAHEDRON LETTERS Bd. 33, Nr. 37, 1992, OXFORD Seiten 5335 - 5338 G. JAHNE ET AL. 'PREPARATION OF CARBOCYCLIC PHOSPHONATENUCLEOSIDES'
- J.Chem.Soc.Chem.Commun.1981,1089

## Beschreibung

Die vorliegende Erfindung betrifft ein Verfahren zur Herstellung von in 5-Stellung substituierten Cytosinen und anderen 4,5-disubstituierten Pyrimidin-2(1H)-onen, sowie dabei auftretende Zwischenprodukte.

Die Alkylierung von heterocyclischen stickstoffhaltigen Basen wie Uracil, Thymin und anderer in 5-Stellung substituierter Uracile liefert im allgemeinen ein schwer auftrennbares Gemisch der an N1 und N3 monosubstituierten zusammen mit den an N1 und N3 doppelt substituierten Produkte (siehe z.B. J.L.Rabinowitz und S.Gurin: J.Am.Chem.Soc. 75, 5758 (1953); S. Kamata, N. Haga, T.Matsui und W.Nagata: Chem.Pharm.Bull. 33, 3160 (1985); N.Ueda, T.Kawabata und K.Takemoto: J.Heterocyclic Chem. 8, 827 (1971)).
Die vergleichbare Reaktion mit Cytosin dagegen erfolgt mit hoher Regioselektivität an N1.
Es ist bekannt, daß an N1 alkylierte Uracilderivate wie z. B. Triacetyluridin oder Diacerylthymidin über die Umwandlung in die 4-(3-Nitro-1,2,4-triazol-1-yl)- oder die 4-(1,2,4-Triazol-1-yl)-Derivate in die entsprechenden 4-Amino-,
4-Alkylamino-,4-Dialkylamino- oder 4-Arylamino-Verbindungen umgewandelt werden können (siehe z. B. K.J.Divakar, C.B.Reese: J. Chem. Soc. Perkin Trans. I, 1982, 1171; W. L. Sung: J. Chem. Soc. Chem.Commun., 1981, 1089).
Ferner ist bekannt, daß sich Cytosinderivate mittels Reaktion mit Natriumnitrit und Säure in Uracilderivate umwandeln lassen.

Somit ließen sich durch selektive Alkylierung an N1 von Cytosin bzw. von in 5-Stellung substituierten Cytosinen und nachfolgendem Umsatz mit Natriumnitrit/Saure an N1 alkylierte Uracile bzw. an N1 alkylierte und in 5-Stellung substituierte Uracile darstellen.

Aus PL 115,858 ist weiterhin bekannt, daß sich aus 5-Fluorouracil in einer dreistufigen Synthese 5-Fluorocytosin herstellen läßt.

Es wurde nun überraschenderweise gefunden, daß sich auch in N1-Stellung nicht alkyliertes Uracil und in N1-Stellung nicht alkylierte in 5-Stellung substituierte Uracile durch ein einfaches Verfahren in die entsprechenden in Position 4 mit Sauerstoff, Schwefel oder Stickstoff substituierten Pyrimidine überführen lassen.

Erfindungsgegenstand ist demzufolge ein Verfahren zur Herstellung von Pyrimidinen der Formel I 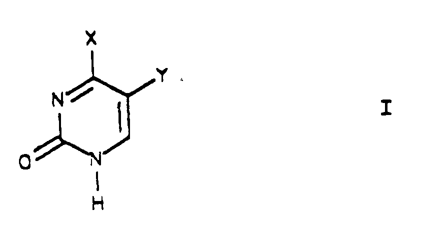
wobei X Amino, Alkylamino, Dialkylamino, Hydrazino, N2-Alkylhydrazino, N2-Dialkylhydrazino, Alkenylamino, Alkinylamino, Benzylamino (gegebenenfalls ringsubstituiert), Dialkenylamino, Dialkinylamino, Dibenzylamino (gegebenenfalls ringsubstituiert), Phenylamino (gegebenenfalls ringsubstituiert), Alkoxy, Alkenyloxy, Alkinyloxy, Benzyloxy (gegebenenfalls ringsubstituiert), Phenyloxy (gegebenenfalls ringsubstituiert), Mercapto, Alkylthio, Alkenylthio, Alkinylthio, Phenylthio (gegebenenfalls ringsubstituiert) oder Benzylthio (gegebenenfalls ringsubstituiert)
und Y Wasserstoff, C1 - C6 - Alkyl, C1 - C5 - Cycloalkyl, Benzyl (gegebenenfalls ringsubstituiert), Benzyloxymethyl (gegebenenfalls ringsubstituiert), Halogen, Ethenyl, Ethinyl, (E)-2-Bromvinyl, Vinyl, (E)-2-Alkoxycarbonylvinyl, oder Propargyl bedeuten,
dadurch gekennzeichnet, daß ein Pyrimidin der Formel II 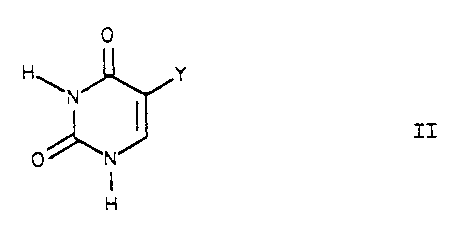 wobei Y die obengenannten Bedeutungen besitzt,
mit einem 5- oder 6-gliedrigen stickstoffhaltigen Heterocyclus und mit einem Phosphorsäurehalogenid in einem aprotischen Lösungsmittel
unter Beifügung einer stickstoffhaltigen Base
zu einem Azolzwischenprodukt der Formel III 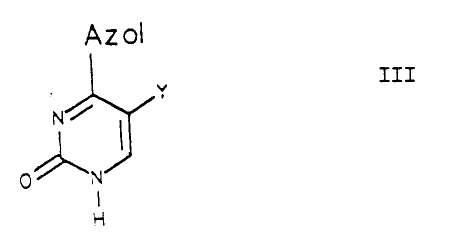 bzw. einem Azinzwischenprodukt der Formel IV, 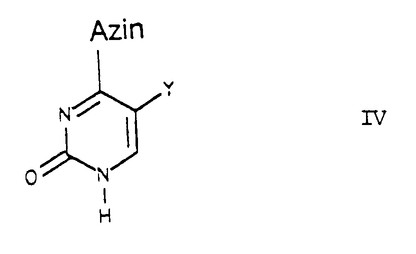 wobei Y die obengenannten Bedeutungen hat, umgesetzt wird, welches als Rohprodukt oder isolierte Zwischenverbindung mit den dem Rest X entsprechenden NH-, OH- oder SH-Nukleophilen (d.h. mit Ammoniak, Alkylamin, Dialkylamin etc.) zu Verbindungen der Formel I, worin X und Y die obengenannten Bedeutungen haben, umgesetzt wird.

Besondere Bedeutung hat das erfindungsgemäße Verfahren zur Herstellung von Pyrimidinen der Formel I,
A) wobei X Amino, Alkylamino, Dialkylamino, Hydrazino, Benzylamino, Alkoxy, Benzyloxy, Mercapto, Alkylthio oder Benzylthio
   und
   Y Wasserstoff, C1 - C6 - Alkyl, Benzyl, Benzyloxymethyl, Fluor, Chlor, Brom, (E)-2-Bromvinyl, Ethinyl oder Propargyl bedeuten.
   Ganz besondere Bedeutung hat das erfindungsgemäße Verfahren zur Herstellung von Pyrimidinen der Formel I,
B) wobei X Amino, Alkylamino, Dialkylamino oder Benzylamino, insbesondere Amino und Y C1 - C6 - Alkyl, Fluor oder Chlor, insbesondere Methyl, bedeuten. Die dabei auftretenden Zwischenprodukte der Formel IIIa, 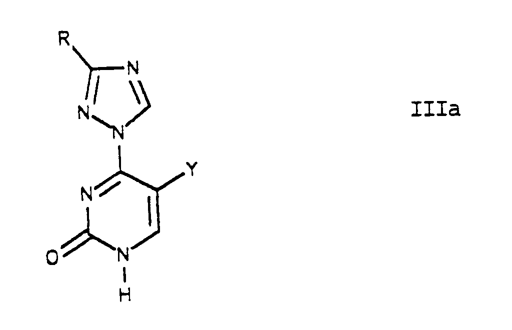 in der R Wasserstoff oder Nitro bedeutet oder der Triazolrest durch einen N-Methylimidazolrest ersetzt ist und in der Y die unter B) genannten Bedeutungen hat, gehören ebenfalls zum Gegenstand der vorliegenden Erfindung.

Insbesondere eignet sich das erfindungsgemäße Verfahren zur Herstellung von 5-Methyl-cytosin (Verbindung der Formel I, worin X Amino und Y Methyl ist). Das dabei auftretende Zwischenprodukt der Formel IIIb 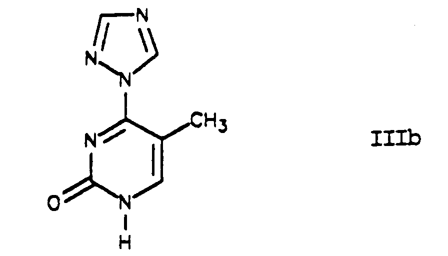 gehört ebenfalls zum Gegenstand der vorliegenden Erfindung.

Die als Substituenten bzw. als Substituenten von Substituenten (z.B. in den Substituenten Alkylamino, Alkoxy etc.) genannten Alkyl-, Alkenyl- und Alkinylgruppen können geradkettig, verzweigt oder cyclisch sein. Sie enthalten vorzugsweise bis zu 8-C-Atome, besonders bevorzugt bis zu 6, insbesondere bis zu 3-C Atome.

Geeignete Alkylgruppen sind beispielsweise Methyl, Ethyl, Propyl, Isopropyl oder Cyclopentyl;
geeignete Alkenylgruppen sind beispielsweise Propenyl, 1-Isobuten-3-yl oder 1-Cyclopenten-3-yl;
geeignete Alkinylgruppen sind beispielsweise 1-Propin-3-yl oder 1-Butin-4-yl.
Die oben angegebenen aromatischen Reste der Substituenten X und Y können ringsubstituiert sein, z. B. mit C1-C5-Alkyl, Nitro, Halogen oder C1-C5-Alkoxy, vorzugsweise mit C1-C3-Alkyl, Chlor oder C1-C3-Alkoxy.
Der obengenannte 5- oder 6-gliedrige stickstoffhaltige Heterocyclus ist vorzugsweise 1,2,4-Triazol oder 3-Nitro-1,2,4-Triazol, insbesondere 1,2,4-Triazol.

Das zur Reaktion benutzte Phosphorsäurehalogenid ist vorzugsweise ein Phosphorsäurechlorid; besonders bevorzugt sind Phosphorsäuretrichlorid und Phosphorsäurediphenylesterchlorid.
Das für die Reaktion geeignete aprotische Lösungsmittel ist vorzugsweise Methylenchlorid, Chloroform, 1,2-Dichlorethan, Acetonitril oder Dioxan.

Die stickstoffhaltige Base, die dem Reaktionsgemisch beigefügt wird, ist vorzugsweise ein C1-C6-Trialkylamin, besonders bevorzugt ein C1-C3-Trialkylamin, insbesondere Triethylamin.

Geeignete stöchiometrische Verhältnisse der Reaktanden sind z. B. 0,15-0,5 Äquivalente des Pyrimidins der Formel II auf 0,9-1,3 Äquivalente des stickstoffhaltigen Heterocyclus mit 0,3-1,1 Äquivalenten Phosphorsäurehalogenid und 0,8-1,5 Äquivalente der stickstoffhaltigen Base. Besonders bevorzugt sind 0,15-0,5 Äquivalente des Pyrimidins der Formel II, ein Äquivalent des stickstoffhaltigen Heterocyclus, 0,33-0,99 Äquivalente Phosphorsäurehalogenid und 1,5 Äquivalente der stickstoffhaltigen Base.

Die Reaktion findet zweckmäßigerweise bei Temperaturen zwischen 0 und 90° C statt und ist nach etwa 2-48 Stunden beendet.

Das erfindungsgemäße Verfahren wird durch nachfolgende Beispiele sowie durch die Patentansprüche näher erläutert:

### Beispiel 1:

### Herstellung des 1,2,4-Triazol-1-yl-Zwischenproduktes der Formel III b (Phosphorsäuretrichlorid-Methode):

Unter Schutzgas (Stickstoff) werden zu einer Suspension von 69 g (1 Mol) 1,2,4-Triazol in 800 ml trockenem Acetonitril bei einer Innentemperatur von 0 - 50°C 50.6 g (0.33 Mol) Phosphorsäuretrichlorid gegeben. Sodann werden über einen Zeitraum von 1 Stunde 101.2 g (1 Mol) Triethylamin zugetropft. Nach weiterem 40minütigem Rühren bei Raumtemperatur werden 18.9 g (0.15 Mol) Thymin zugegeben. Die Suspension wird 24 Stunden bei Raumtemperatur gerührt, mit 25 ml Wasser versetzt, weitere 10 Minuten gerührt und danach filtriert. Der gelbe Rückstand wird in 400 ml Wasser suspendiert, gerührt und nach 1 Stunde abgesaugt. Man erhält 1.72 g 5-Methyl-4-(1,2,4-triazol-1-yl)-pyrimidin-2(1H)-on als gelbliches Pulver mit dem Schmelzpunkt 258 - 264°C.
Die Acetonitrillösung wird eingeengt, der Rückstand in 400 ml Wasser suspendiert, gerührt und abgesaugt. Auf diese Weise werden weitere 7.21 g der Verbindung erhalten.
Die Titelverbindung kann aus Dimethylformamid umkristallisiert werden und schmilzt dann bei 275 - 280°C. 1H NMR (60 MHz, d6-DMSO) δ[ppm]: ca. 12.0 (s, breit, 1H), 9.33 (s, 1H), 8.40 (s, 1H), 8.13 (s, 1H), 2.3 (s, 3H).
Aus den Mutterlaugen lassen sich nach chromatographischer Auftrennung an Kieselgel mit Dichlormethan/Methanol 9/1 als mobile Phase zwei Nebenprodukte isolieren:
Nebenprodukt I: 2,4-Bis(1,2,4-triazol-1-yl)-5-methyl-pyrimidin, farbloses Pulver, Fp.: 193 - 196°C, 1H NMR (270 MHz, d6-DMSO), δ[ppmj: 9.76 (s, 1H), 9.74 (s, 1H), 9.01 (s, 1H), 8.46 (s, 1H), 8.36 (s, 1H), 2.64 (s, 3H).
Nebenprodukt II: 5-Methyl-2-(1,2,4-triazol-4-yl)-4-(1,2,4-triazol-1-yl)-pyrimidin, farblose Kristalle, Fp.: 256 - 261°C, 1H NMR (270 MHz, d6-DMSO), δ[ppm]: 9.92 (s, 1H), 9.52 (s, 2H), 8.99 (s, 1H), 8.46 (s, 1H), 2.63 (s, 3H).
Beide Verbindungen lassen sich durch Behandlung mit 1N Natronlauge (6 Stunden, 30 - 50°C) in das 5-Methyl-4-(1,2,4-triazol-1-yl)-pyrimidin-2(1H)-on überführen.

### Beispiel 2:

### Herstellung des 1,2,4-Triazol-1-yl-Zwischenproduktes der Formel III b (Phosphorsäurediphenylester-chlorid-Methode):

Unter Schuzgas (Argon) werden 69 g (1 Mol) 1,2,4-Triazol in 800 ml trockenem Acetonitril suspendiert. Bei 0 - 10°C werden dieser Suspension unter Rühren 251 g (0.95 Mol) Phosphorsäurediphenylester-chlorid zugefügt. Anschließend werden während einer Stunde 151.79 g (1.5 Mol) Triethylamin zugetropft bevor 63 g (0.5 Mol) Thymin zugegeben werden. Die resultierende Suspension wird 12 Stunden bei Raumtemperatur gerührt. Schließlich wird die Mischung 5 Stunden auf 60 - 80°C erwärmt.Die abgekühlte Suspension wird mit 40 ml Methanol versetzt und 2 Stunden bei Raumtemperatur gerührt. Die Suspension wird filtriert; der Rückstand wird mit einem Liter Diisopropylether verrührt, erneut abgesaugt und mit 800 ml Wasser verrührt. Die wässrige Suspension wird filtriert und der feste Rückstand wird aus 1300 ml Dimethylformamid umkristallisiert. Man erhält 28.6 g 5-Methyl-4-(1,2,4-triazol-1-yl)pyrimidin-2(1H)-on vom Schmelzpunkt 275 - 280°C.

### Beispiel 3:

### Herstellung von 5-Methyl-cytosin (Verbindung der Formel I, worin X Amino und Y Methyl ist) aus einer Verbindung der Formel III b:

1.06 g (6 mMol) 5-Methyl-4-(1,2,4-triazol-1-yl)-pyrimidin-2(1H)-on werden in 30 ml konzentriertem wässrigen Ammoniak gelöst und 3 Stunden bei Rückflußtemperatur gerührt. Die abgekühlte Lösung wird vollständig eingeengt, heiß in Wasser gelöst, abgekühlt und mit der dreifachen Menge Aceton versetzt. Der Niederschlag wird abgesaugt und im Vakuum getrocknet. Man erhält 0.73 g (97.3 % d. Th.) 5-Methyl-cytosin als farbloses Pulver vom Schmelzpunkt 271 - 274°C.

### Beispiel 4:

### Herstellung von N4-Methyl-5-methyl-cytosin (Verbindung der Formel I, worin X Methylamino und Y Methyl ist) aus einer Verbindung der Formel III b:

1.06 g (6 mMol) 5-Methyl-4-(1,2,4-triazol-1-yl)-pyrimidin-2(1H)-on werden in 20 ml 40%iger wäßriger Methylaminlösung gelöst und 5 Stunden bei Raumtemperatur gerührt. Die Lösung wird eingeengt und über Kieselgel mit Essigsäureethylester/Methanol 2/1 chromatographiert. Man erhält 0.67 g (80.3% d. Th.) N4-Methyl-5-methyl-cytosin als farbloses Pulver mit einem Schmelzpunkt von 160 - 165°C (Zers.). 1H NMR, d6-DMSO), δ[ppm]: 10.4 (s, 1H), 7.17 (s, 1H), 7.1 (s, breit, 1H), 2.8 (d, 3H), 1.82 (d, 3H).

### Beispiel 5:

### Herstellung von 4-Methoxy-5-methyl-pyrimidin-2(1H)-on (Verbindung der Formel I, worin X Methoxy und Y Methyl ist) aus einer Verbindung der Formel III b:

1.06 g (6 mMol) 5-Methyl-4-(1,2,4-triazol-1-yl)-pyrimidin-2(1H)-on werden mit 972 mg (18 mMol) Natriummethylat in 30 ml wasserfreiem Methanol 4 Stunden unter Feuchtigkeitsausschluß unter Rückfluß gekocht. Die abgekühlte Lösung wird mit Essigsäure neutralisiert, eingeengt und an Kieselgel mit Essigsäureethylester/Methanol 9/1 chromatographiert. Man erhält 770 mg (91.7% d. Th.) 4-Methoxy-5-methyl-pyrimidin-2(1H)-on mit dem Schmelzpunkt 196 - 199°C. 1H NMR (270 MHz, d6-DMSO), δ[ppm]: 11.09 (s, 1H), 7.51 (d, 1H), 3.83 (s, 3H), 1.85 (s, 3H).

### Beispiel 6:

### Herstellung von 4-Isopropoxy-5-methyl-pyrimidin-2(1H)-on (Verbindung der Formel I, worin X Isopropoxy und Y Methyl ist) aus einer Verbindung der Formel III b:

1.06 g (6 mMol) 5-Methyl-4-(1,2,4-triazol-1-yl)pyrimidin-2(1H)-on werden zu einer Lösung von 1.476 g (18 mMol) Natriumisopropylat in 30 ml wasserfreiem Isopropanol gegeben und 4 Stunden bei 50°C gerührt. Die erhaltene Lösung wird unter Kühlung mit Essigsäure neutralisiert, eingeengt und an Kieselgel mit Dichlormethan/Methanol 9/1 chromatographiert. Das Eluat wird eigeengt, mit Ether verrührt, und man erhält als Rückstand 0.71 g (70.4% d. Th.) 4-Isopropoxy-5-methyl-pyrimidin-2(1H)-on mit dem Schmelzpunkt 206 - 207°C. 1H NMR (270 MHz, d6-DMSO), δ[ppm]: 11.02 (s, 1H), 7.48 (s, 1H), 5.25 (m, 1H), 1.81 (s, 3H), 1.27 (d, 6H).

### Beispiel 7:

### Herstellung von 4-Benzyloxy-5-methyl-pyrimidin-2(1H)-on (Verbindung der Formel I, worin X Benzyloxy und Y Methyl ist) aus einer Verbindung der Formel III b:

Zu einer Lösung von 2.34 g (18 mMol) Natriumbenzylat in 25 ml trockenem Benzylalkohol werden 1.06 g (6 mMol) 5-Methyl-4-(1, 2, 4-trazol-1-yl)pyrimdin-2(1H)-on und 25 ml trockenes Dimethylformamid zugegeben. Die Mischung wird 2 Stunden unter Rühren auf 100°C erwärmt. Die abgekühlte Lösung wird mit Essigsäure neutralisiert, eingeengt und über Kieselgel mit Essigsäureethylester/Methanol 9/1 chromatographiert. Man erhält 880 mg (67.9% d. Th.) 4-Benzyloxy-5-Methyl-pyrimidin-2(1H)-on mit dem Schmelzpunkt 181 - 182°C. 1H NMR (270 MHz, d6-DMSO), δ[ppm]: 11.14 (s, 1H), 7.56 (d, 1H), 7.41 (m, 5H), 5.35 (s, 2H), 1.87 (s, 3H).

### Beispiel 8:

### Herstellung von 4-Ethylthio-5-methyl-pyrimidin-2(1H)-on (Verbindung der Formel I, worin X Ethylthio und Y Methyl ist) aus einer Verbindung der Formel III b:

0.7 ml (9 mMol) Ethylmercaptan werden in 25 ml trockenem Dimethylformamid bei 0°C mit 786 mg (18 mMol) einer 55%igen Natriumhydridemulsion versetzt und 30 Minuten bei 0°C gerührt. Sodann werden 1.06 g (6 mMol) 5-Methyl-4-(1,2,4-triazol-1-yl)-pyrimidin-2(1H)-on zugegeben und die Mischung wird 3 Stunden bei Raumtemperatur gerührt. Die Reaktionsmischung wird durch Zugabe von Essigsäure neutralisiert, eingeengt und über Kieselgel mit Dichlormethan/Methanol 20/1 chromatographiert. Man erhält 0.64 g (62.7% d. Th.) 4-Ethylthio-5-methyl-pyrimidin-2(1H)-on mit dem Schmelzpunkt 181 - 184°C. 1H NMR (60 MHz, d6-DMSO), δ[ppm]: 11.45 (s, 1H), 7.55 (s, 1H), 3.10 (q, 2H), 1.90 (s, 3H), 1.25 (t, 3H).

## Patentansprüche

1. Verfahren zur Herstellung von Pyrimidinen der Formel I, 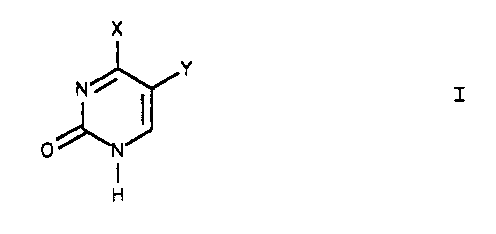
wobei X Amino, Alkylamino, Dialkylamino, Hydrazino, N2-Alkylhydrazino, N2-Dialkylhydrazino, Alkenylamino, Alkinylamino, Benzylamino (gegebenenfalls ringsubstituiert), Dialkenylamino, Dialkinylamino, Dibenzylamino (gegebenenfalls ringsubstituiert), Phenylamino (gegebenenfalls ringsubstituiert), Alkoxy, Alkenyloxy, Alkinyloxy, Benzyloxy (gegebenenfalls ringsubstituiert), Phenyloxy (gegebenenfalls ringsubstituiert), Mercapto, Alkylthio, Alkenylthio, Alkinylthio, Phenylthio (gegebenenfalls ringsubstituiert) oder Benzylthio (gegebenenfalls ringsubstituiert) und
Y Wasserstoff, C1 - C6 - Alkyl, C1 - C5 - Cycloalkyl, Benzyl (gegebenenfalls ringsubstituiert), Benzyloxymethyl (gegebenenfalls ringsubstituiert), Halogen, Ethenyl, Ethinyl, (E)-2-Bromvinyl, Vinyl, (E)-2 Alkoxycarbonylvinyl oder Propargyl bedeuten,
dadurch gekennzeichnet, daß ein Pyrimidin der Formel II 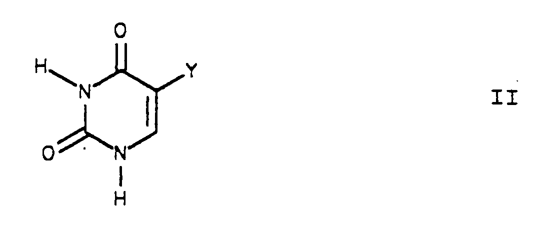 wobei Y die oben genannten Bedeutungen besitzt, mit einem 5- oder 6-gliedrigen stickstoffhaltigen Heterocyclus und mit einem Phosphorsäurehalogenid in einem aprotischen Lösungsmittel unter Beifügung einer stickstoffhaltigen Base zu einem Azolzwischenprodukt der Formel III 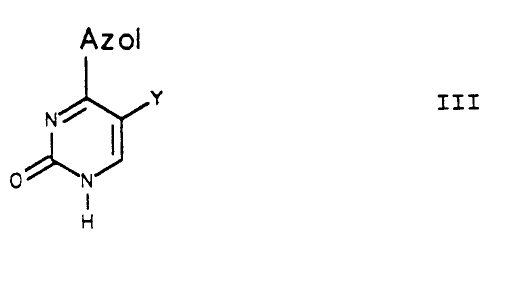 oder einem Azinzwischenprodukt der Formel IV 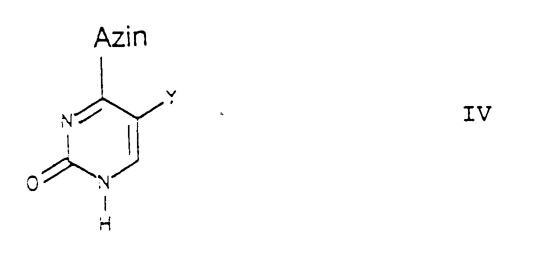 wobei Y die obengenannten Bedeutungen hat, umgesetzt wird, welches als Rohprodukt oder isolierte Zwischenverbindung mit den dem Rest X entsprechenden NH-, OH- oder SH-Nukleophilen zu Verbindungen der Formel I, worin X und Y die obengenannten Bedeutungen haben, umgesetzt wird.

2. Verfahren gemäß Anspruch 1, dadurch gekennzeichnet, daß in den Verbindungen der Formel I,
X Amino, Alkylamino, Dialkylamino, Hydrazino, Benzylamino, Alkoxy, Benzyloxy, Mercapto, Alkylthio oder Benzylthio und
Y Wasserstoff, C1 - C6 - Alkyl, Benzyl, Benzyloxymethyl, Fluor, Chlor, Brom, (E)-2-Bromvinyl, Ethinyl oder Propargyl bedeuten.

3. Verfahren gemäß den Ansprüchen 1 oder 2, dadurch gekennzeichnet, daß in den Verbindungen der Formel I,
X Amino, Alkylamino, Dialkylamino oder Benzylamino
und Y C1 - C6 - Alkyl, Fluor oder Chlor bedeuten.

4. Verfahren gemäß den Ansprüchen 1 bis 3, dadurch gekennzeichnet, daß in der Verbindung der Formel I
X Amino und Y Methyl ist.

5. Verfahren gemäß einem oder mehreren der Ansprüche 1 - 4, dadurch gekennzeichnet, daß das Phosphorsäurehalogenid Phosphorsäuretrichlorid oder Phosphorsäurediphenylesterchlorid ist.

6. Verfahren gemäß einem oder mehreren der Ansprüche 1 - 5, dadurch gekennzeichnet, daß der stickstoffhaltige Heterocyclus 1,2,4-Triazol oder 3-Nitro-1,2,4-Triazol, ist.

7. Verfahren gemäß einem oder mehreren der Ansprüche 1 - 6, dadurch gekennzeichnet, daß 0,15-0,5 Äquivalente des Pyrimidins der Formel II mit 0,9-1,3 Äquivalenten des stickstoffhaltigen Heterocyclus mit 0,3-1,1 Äquivalenten Phosphorsäurehalogenid und 0,8 - 1,5 Äquivalenten der stickstoffhaltigen Base umgesetzt werden.

8. Verbindungen der Formel IIIa 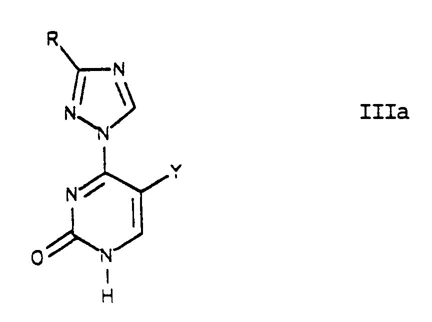 wobei R Wasserstoff oder Nitro bedeutet und wobei Y C1-C6-Alkyl, Fluor oder Chlor bedeutet.

9. Verbindung gemäß Anspruch 8, dadurch gekennzeichnet, daß sie die Formel IIIb hat

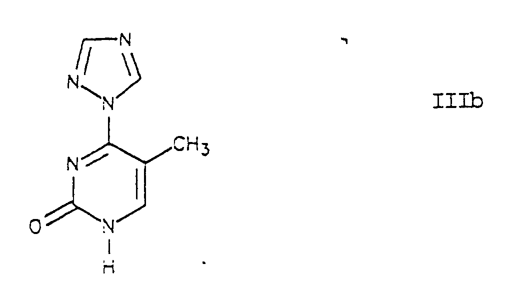

## Claims

1. A process for the preparation of a pyrimidine of the formula I, 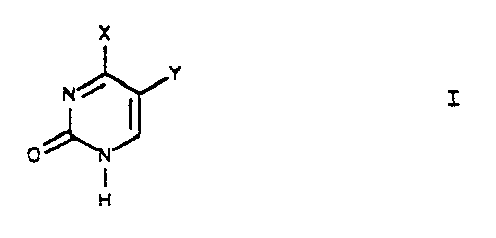
where X is amino, alkylamino, dialkylamino, hydrazino, N2-alkylhydrazino, N2-dialkylhydrazino, alkenylamino, alkynylamino, benzylamino (which may be ring-substituted), dialkenylamino, dialkynylamino, dibenzylamino (which may be ring-substituted), phenylamino (which may be ring-substituted), alkoxy, alkenyloxy, alkynyloxy, benzyloxy (which may be ring-substituted), phenyloxy (which may be ring-substituted), mercapto, alkylthio, alkenylthio, alkynylthio, phenylthio (which may be ring-substituted) or benzylthio (which may be ring-substituted)
and
Y is hydrogen, C₁-C₆-alkyl, C₁-C₅-cycloalkyl, benzyl (which may be ring-substituted), benzyloxymethyl (which may be ring-substituted), halogen, ethenyl, ethynyl, (E)-2-bromovinyl, vinyl, (E)-2-alkoxy-carbonylvinyl or propargyl,
which comprises reacting a pyrimidine of the formula II 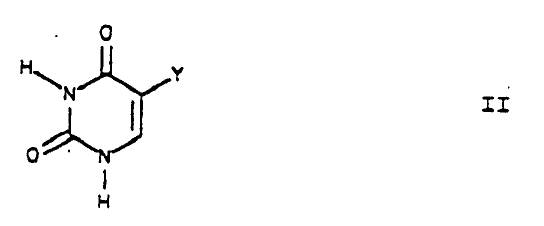 where Y has the abovementioned meanings, with a 5-or 6-membered nitrogen-containing heterocycle and a phosphoric acid halide in an aprotic solvent with addition of a nitrogen-containing base to give an azole intermediate of the formula III 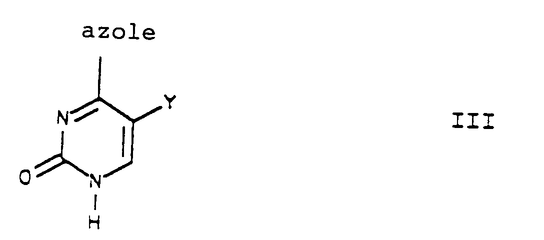 or an azine intermediate of the formula IV 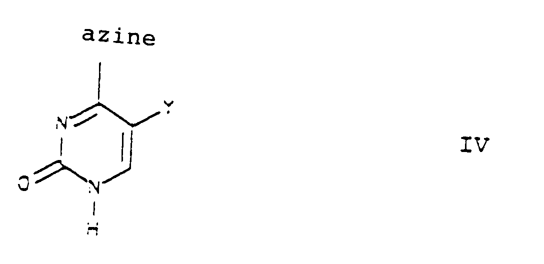 where Y has the abovementioned meanings, which intermediate is reacted as a crude product or isolated intermediate compound with the NH, OH or SH nucleophiles corresponding to the radical X to give compounds of the formula I, in which X and Y have the abovementioned meanings.

2. The process as claimed in claim 1, wherein, in the compounds of the formula I,
X is amino, alkylamino, dialkylamino, hydrazino, benzylamino, alkoxy, benzyloxy, mercapto, alkylthio or benzylthio and
Y is hydrogen, C₁-C₆-alkyl, benzyl, benzyloxymethyl, fluorine, chlorine, bromine, (E)-2-bromovinyl, ethynyl or propargyl.

3. The process as claimed in claims 1 or 2, wherein, in the compounds of the formula I,
X is amino, alkylamino, dialkylamino or benzylamino and Y is C₁-C₆-alkyl, fluorine or chlorine.

4. The process as claimed in claims 1 to 3, wherein, in the compound of the formula I,
X is amino and Y is methyl.

5. The process as claimed in one or more of claims 1-4, wherein the phosphoric acid halide is phosphoryl trichloride or diphenyl chlorophosphate.

6. The process as claimed in one or more of claims 1-5, wherein the nitrogen-containing heterocycle is 1,2,4-triazole or 3-nitro-1,2,4-triazole.

7. The process as claimed in one or more of claims 1-6, wherein 0.15-0.5 equivalent of the pyrimidine of the formula II is reacted with 0.9-1.3 equivalents of the nitrogen-containing heterocycle, with 0.3-1.1 equivalents of phosphoric acid halide and 0.8-1.5 equivalents of the nitrogen-containing base.

8. A compound of the formula IIIa 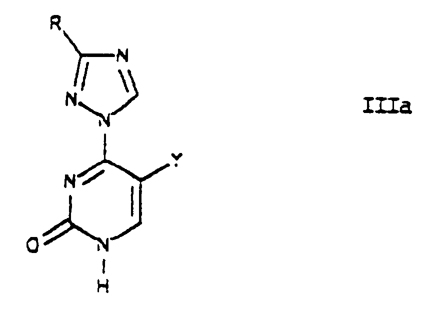 where R is hydrogen or nitro and where Y is C₁-C₆-alkyl, fluorine or chlorine.

9. A compound as claimed in claim 8 which has the formula IIIb

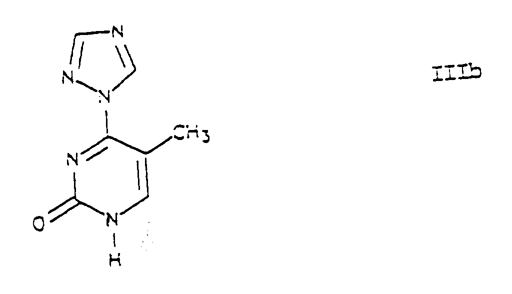

## Revendications

1. Procédé pour la préparation de pyrimidines de formule I 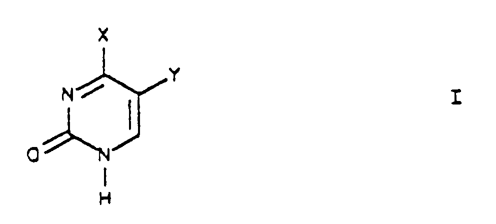
dans laquelle X représente un groupe amino, alkylamino, dialkylamino, hydrazino, N2-alkylhydrazino, N2-dialkyl-hydrazino, alcénylamino, alcynylamino, benzylamino (éventuellement substitué sur le noyau), dialcénylamino, dialcynylamino, dibenzylamino (éventuellement substitué sur le noyau) phénylamino (éventuellement substitué sur le noyau) alcoxy, alcényloxy, alcynyloxy, benzyloxy (éventuellement substitué sur le noyau), phényloxy (éventuellement substitué sur le noyau), mercapto, alkylthio, alcénylthio, alcynylthio, phénylthio (éventuellement substitué sur le noyau) ou benzylthio (éventuellement substitué sur le noyau),
et Y représente un atome d'hydrogène ou d'halogène, ou un groupe alkyle en C₁-C₆, cycloalkyle en C₁-C₅, benzyle (éventuellement substitué sur le noyau), benzyloxyméthyle (éventuellement substitué sur le noyau), éthényle, éthynyle, (E)-2-bromovinyle, vinyle, (E)-2-alcoxycarbonylvinyle ou propargyle,
caractérisé en ce que l'on fait réagir une pyrimidine de formule II 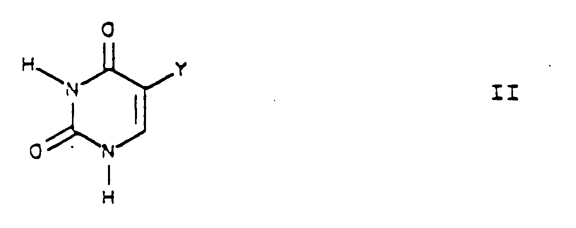 dans laquelle Y a les significations données plus haut, avec un hétérocycle azoté à 5 ou 6 chaînons et avec un halogénure de phosphoryle, dans un solvant aprotique, avec addition d'une base azotée, pour obtenir un produit intermédiaire azole de formule III 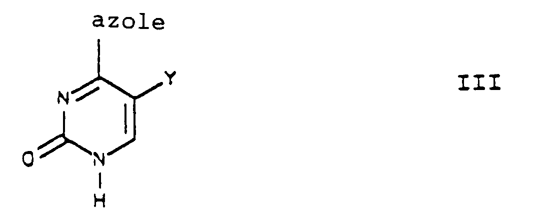 ou à un produit intermédiaire azine de formule IV 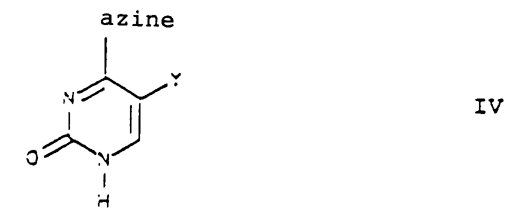 Y ayant les significations données plus haut,
qui est mis en réaction, sous forme de produit brut ou de composé intermédiaire isolé, avec les agents nucléophiles comportant un groupe NH, OH ou SH, correspondant au radical X, pour l'obtention des composés de formule I dans lesquels X et Y ont les significations données plus haut.

2. Procédé selon la revendication 1, caractérisé en ce que, dans les composés de formule I,
X représente un groupe amino, alkylamino, dialkylamino, hydrazino, benzylamino, alcoxy, benzyloxy, mercapto, alkylthio ou benzylthio, et
Y représente un atome d'hydrogène ou de fluor, chlore ou brome, ou un groupe alkyle en C₁-C₆, benzyle, benzyloxyméthyle, (E)-2-bromovinyle, éthynyle ou propargyle.

3. Procédé selon la revendication 1 ou 2, caractérisé en ce que, dans les composés de formule I,
X représente un groupe amino, alkylamino, dialkylamino ou benzylamino,
et Y représente un groupe alkyle en C₁-C₆, ou l'atome de fluor ou de chlore.

4. Procédé selon les revendications 1 à 3, caractérisé en ce que, dans le composé de formule I,
X est le groupe amino et Y est le groupe méthyle.

5. Procédé selon une ou plusieurs des revendications 1 à 4, caractérisé en ce que l'halogénure de phosphoryle est le trichlorure de phosphoryle ou le chlorure de phosphate de diphényle.

6. Procédé selon une ou plusieurs des revendications 1 à 5, caractérisé en ce que l'hétérocycle azoté est le 1,2,4-triazole ou le 3-nitro-1,2,4-triazole.

7. Procédé selon une ou plusieurs des revendications 1 à 6, caractérisé en ce que l'on fait réagir 0,15-0,5 équivalent de la pyrimidine de formule II 0,9-1,3 équivalent de l'hétérocycle azoté et avec 0,3-1,1 équivalent d'halogénure de phosphoryle et 0,8-1,5 équivalent de la base azotée.

8. Composés de formule IIIa 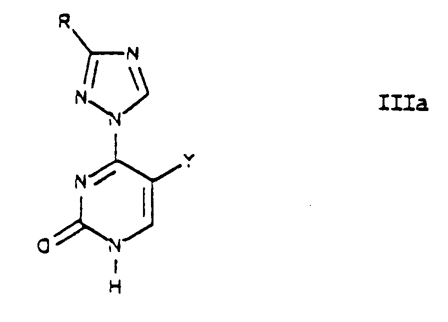 dans laquelle R représente un atome d'hydrogène ou le groupe nitro, et dans laquelle Y représente un groupe alkyle en C₁-C₆ ou l'atome de fluor ou de chlore.

9. Composé selon la revendication 8, caractérisé en ce qu'il correspond à la formule IIIb
